(19) Europäisches Patentamt
European Patent Office
Office européen des brevets

(11) Publication number: **0 102 840**
**B1**

# (12) EUROPEAN PATENT SPECIFICATION

(45) Date of publication of patent specification: **15.10.86**

(51) Int. Cl.⁴: **C 07 C 41/06**, C 07 C 29/04, B 01 J 39/04

(21) Application number: **83305094.1**

(22) Date of filing: **02.09.83**

## (54) Preparation of ethers and alcohols from olefins and conversion of olefins to ethers and alcohols.

(30) Priority: **03.09.82 US 414841**

(43) Date of publication of application:
**14.03.84 Bulletin 84/11**

(45) Publication of the grant of the patent:
**15.10.86 Bulletin 86/42**

(84) Designated Contracting States:
**AT BE CH DE FR GB IT LI LU NL SE**

(56) References cited:
**GB-A-1 381 455**
**GB-A-1 396 488**
**GB-A-2 003 141**
**US-A-2 480 940**
**US-A-3 979 461**
**US-A-4 340 769**

(73) Proprietor: **DUOLITE INTERNATIONAL S.A.**
**107, Rue Edith Cavell**
**F-94400 Vitry-sur-Seine (FR)**

(72) Inventor: **Rothschild, Walter G.**
**521 Mentor Avenue**
**Painesville Ohio 44077 (US)**

(74) Representative: **Oliver, Roy Edward et al**
**POLLAK MERCER & TENCH High Holborn House**
**52-54 High Holborn**
**London WC1V 6RY (GB)**

**EP 0 102 840 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to the preparation of ethers and/or alcohols from olefins and to the conversion of ethers and/or alcohols to olefins, the olefins having a double bond attached to tertiary carbon atom, and the preparation or conversion being carried out in the presence of a sulphonated strong acid exchange resin catalyst.

Recent concern with environmental pollution by lead from the exhaust gases of internal combustion engines has forced a transition away from the use of lead antiknock compounds in gasoline. In order to produce unleaded gasoline having acceptable octane value without varying the compound ratio of the gasoline, it has become necessary to use organic blending compounds with high octane ratings.

A variety of organic compounds are known as octane value improving agents. Particularly well known are the ethers and alcohols having branched alkyl groups, such as methyl tert-butyl ether (MTBE), ethyl tert-butyl ether, isopropyl tert-butyl ether, tert-amyl methyl ether (TAME) and tert-butyl alcohol (TBA). The preparation of these ethers and alcohols by the catalytic addition of an alcohol or water to an olefin having a double bond on a tertiary carbon atom has been extensively studied. Examples of such olefins are isobutylene, 2-methyl butene-1, 2-methyl butene-2, 2-methyl pentene-1 and 2-methyl pentene-2. The state of the art in this field may be ascertained by reference to U.S. Patent Nos. 2,480,940; 3,037,052; 3,119,766; 3,940,450; 3,979,450; 3,979,461; 4,096,194; 4,219,678; 4,267,393; 4,310,710; and 4,316,724; the disclosures of which are incorporated herein by reference.

In general, the catalysts employed for the etherification or alcoholification of olefins have been acids such as $H_2SO_4$, Lewis acids, platinum metal salts, and various heterogeneous catalysts. More recently the solid sulphonated organic resins, originally developed for ion exchange applications, have been used to advantage (e.g. U.S. Patent No. 2,480,940). This group of resins includes sulphonated polystyrene resins cross-linked with divinylbenzene which can have gel-like characteristics (U.S. Patent No. 3,119,766) or which can exhibit a sponge structure (U.S. Patent No. 3,037,052). The large hydrogen ion concentration provided by the strongly acidic sulfonated cation exchange resins promotes a faster reaction rate than the previously used liquid catalysts, but the resins suffer from the disadvantage of displaying variations in catalytic activity.

The present invention provides a process of preparation of ethers and/or alcohols from olefins or of conversion of ethers and/or alcohols to olefins, the olefins having a double bond attached to a tertiary carbon atom, in the presence of a sulphonated strong acid ion exchange resin catalyst, characterized in that the cation exchange resin catalyst has a pore volume in the range from 0.50 to 0.70 $cm^3/g$. The use of a catalyst resin having these specific porosity characteristics results in unexpectedly improved reaction kinetics, with the additional advantage that the resins themselves have better physical properties.

Although the invention will be described with specific reference to the preparation of MTBE for the purpose of clarity, it should be understood that other ethers and alcohols prepared by the catalytic addition of alcohol or water to olefins having a double bond at the tertiary carbon are within the scope of the invention.

MTBE is synthesized according to the following exothermic reaction:

$$\begin{array}{ccc} CH_3 & & CH_3 \\ | & & | \\ C{=}CH_2 + CH_3OH & \xrightarrow{H^{\cdot}} & CH_3{-}C{-}O{-}CH_3 \\ | & & | \\ CH_3 & & CH_3 \end{array}$$

and is an example of an acid catalyzed alcohol addition reaction. It is reversible and the equilibrium is a function of temperature. Therefore, there exists a classical equilibrium-rate conflict, i.e., at low temperatures MTBE is favored in the equilibrium, but the reaction rate is low, and vice versa.

A typical process for the preparation of MTBE uses an isobutylene (isobutene) containing hydrocarbon and methanol as the starting materials. Pure isobutylene may of course be used, but hydrocarbon mixtures containing 10 to 50 percent isobutylene are also useful. Such mixtures may contain, in addition to isobutylene, n-butane, isobutane, butene-1 and butene-2. For example, the $C_4$ fractions which are obtained by thermal cracking, steam cracking and catalytic cracking can be conveniently used. Commercially available methanol is satisfactory for use in the process.

The reactants are introduced into a suitable reactor, typically of the fixed bed shell and tube type, or a fluidized bed. Methanol is typically fed in stoichiometric excess. In passing through the catalyst bed of strongly acid cation exchange resin, the isobutylene and methanol are heated by the strongly exothermic reaction. The $C_4$ hydrocarbons other than isobutylene pass through the catalyst bed unreacted. Operating temperatures between 30° and 90°C are common with a pressure (usually about 4.1 to 17.2 bars (about 60 to 250 psi)) appropriate to maintain the $C_4$ hydrocarbons in a liquid state. The size of the reactor and the catalyst bed is not restricted. The product MTBE and unreacted methanol and $C_4$ hydrocarbons are separated downstream of the reactor by distillation.

It is of course possible to use a plurality of reactors arranged in series or parallel relationship, and to operate in either a batch or continuous mode. In practice, two reactors are often used, with the first

operated at a higher temperature to bring the reaction to near 90 percent conversion quickly. The second reactor then operates at a lower temperature to allow the equilibrium to reach a higher conversion.

While not wishing to be bound by theoretical considerations, the following mechanism appears to explain the catalytic addition of methanol to isobutylene to form MTBE. The catalysts generally used for the preparation of MTBE were initially made as ion exchange resins to remove cations from solution by exchanging a hydrogen ion for the cation. In the MTBE reaction, the primary function of the catalyst seems to be to donate a hydrogen ion to the isobutylene (protonation) to form a tertiary carbonium ion. Methanol then acts as a nucleophile and attacks the carbonium ion to form the ether and another hydrogen ion. Isobutylene is also a nucleophile, although not as strong as methanol, and can react with the carbonium ion to form a dimer. Methanol is therefore used in excess to suppress the isobutylene dimer formation. Water, if present, is a better nucleophile than methanol and will form tert-butyl alcohol.

Batch rate studies indicate that the reaction is first order with respect to isobutylene and independent of catalyst and methanol concentrations. This coincides with the proposed mechanism and implies that the rate determining step is the protonation of isobutylene to form the carbonium ion. The attack of the nucleophile, methanol, is much quicker. If isobutylene is in stoichiometric excess the rate is dependent on methanol, but the MTBE reaction is not normally run under these conditions.

The catalysts conventionally used in etherification and alcoholification reactions have been the strong acid cation exchange resins. Such resins are represented by sulfonated condensation polymers of phenol and formaldehyde, and by sulfonated copolymers of styrene and divinylbenzene. The more widely used styrene-divinyl-benzene copolymers are prepared by cross-linking styrene with divinylbenzene, and then treating the resulting copolymer with a sulfonating agent to introduce sulfonic acid groups into the matrix. Both types of resin can be prepared in a gel form or in a macroporous, sponge-like form. Typical strong acid styrene-divinylbenzene resins are sold under the trademarks Duolite C20, Duolite C26, Amberlyst 15, Amberlite IR-120, Amberlite 200 and Dowex 50.

As is well known in the art, the physical properties of the sulfonated strong acid cation exchange resins may be varied over a wide range by control of three key variables in the production of the base copolymers. These factors are the degree of cross-linking, the nature of the inert diluent, or porogen, used in the polymerization reaction, and the amount of porogen used relative to the monomers. Control of these variables allows the preparation of catalyst resins having characteristics such as surface area, porosity, strength, degree of swelling and shrinkage, and exchange capacity defined within a desired range.

It has now been discovered that if the pore volume of the catalyst resins is maintained within a specific range, there is an unexpected improvement in the kinetics of etherification and alcoholification reactions using the catalyst. More specifically, if the pore volume of the catalyst resin is in the range from 0.50 to 0.70 $cm^3/g$, the reaction kinetics are dramatically improved. Use of a catalyst having these characteristics can typically improve the kinetics of an MTBE preparation reaction by 65 percent or more.

The catalysts have the additional advantage of reduced swelling in the wet state. This is probably due to the higher cross-linking generally found in base copolymers used to prepare resins having the desired pore volume. A resin which expands less in contact with water or alcohol provides a more physically stable catalyst, fewer reactor loading problems and reduced pressure drop across the catalyst bed.

The invention is more fully described in the following specific examples.

Example 1 (for comparison)

Preparation of MTBE using conventional catalyst

A one gallon autoclave reactor equipped with agitator, external electrical heating coils and internal water cooling coils was charged with 460 ml methanol (ACS grade). Next 1179 g of a $C_4$ mixture containing 45 percent isobutylene, 20 percent butene-1 and 35 percent isobutane by weight was introduced into the reactor. This resulted in a stoichiometric ratio of 1.2:1.0 methanol to isobutylene. The reactor was then brought to a temperature of 70°C and the agitator was turned on. The catalyst, in this instance 41.4 g of dry Amberlyst 15 (Rohm and Haas Company) was weighed into an injection tube connected to the reactor, and was blanketed with nitrogen at about 5.2 bars (75 psi) above the reactor pressure. The amount of dry catalyst was calculated to be equivalent to 119 ml of wet resin. To initiate the reaction, the catalyst resin was injected into the reactor. The reactor temperature was maintained at 70°C throughout, and samples were taken periodically to determine percent conversion of isobutylene versus time. Samples were taken by means of a double valved sampling dip tube and were analyzed by gas chromatography.

Results are set forth in Table I.

Example 2

Preparation of MTBE by the process of the invention

The procedure set forth in Example 1 was followed, using the same apparatus. The catalyst, designated Duolite ES-276-E, was provided by Duolite International. It was a sulfonated strong acid cation exchange resin based on a styrene-divinylbenzene copolymer prepared by conventional suspension polymerization in the presence of a nonpolymerizable porogen. The catalyst resin had a pore volume of 0.67 $cm^3/g$. The reactor was charged with 42.3 g of dry catalyst, equivalent to 119 ml of wet resin. The reaction rate was followed as in Example 1, and the results are set forth in Table I.

TABLE I

Percent conversion to MTBE with time (hours)

| | 0 | .25 | .5 | .75 | 1.0 | 1.5 | 2.0 | 2.5 | 3.0 |
|---|---|---|---|---|---|---|---|---|---|
| Example 1 | 0 | 33 | 57 | 73 | 80 | 86.5 | 90 | 92 | 94 |
| Example 2 | 0 | 48 | 72 | 83 | 89 | 92 | 93 | 94 | 94 |

In order to compare the reaction kinetics for the foregoing catalysts and additional cation exchange resins, rate constants were derived by fitting the kinetic data to a rate expression. Larger rate constants are indicative of better catalysts. Under typical operating conditions the rate of formation of MTBE was found to be independent of catalyst and methanol concentration, and first order with respect to isobutylene. The MTBE reaction is reversible and thus suggests the expression

$$r=k_{FOR}C_{IB}-k_{REV}C_{MTBE}$$

where $k_{FOR}$ and $k_{REV}$ represent the forward and reverse rate constant (a function of temperature), and $C_{IB}$ and $C_{MTBE}$ represent the concentrations of isobutylene and methyl tert-butyl ether. The concentrations of isobutylene and MTBE can be put in terms of the fraction of isobutylene converted (X), and the initial isobutylene concentration (Co), as

$$C_{IB}=Co(1-X)$$

$$C_{MTBE}=CoX.$$

With substitution and rearrangement with $K=k_{REV}/k_{FOR}$, the expression becomes

$$r=k_{FOR}Co(1-X-KX).$$

At equilibrium conditions the reaction rate is zero and K becomes

$$K=\left(\frac{1}{X_{eq}}\right)-1$$

where $X_{eq}$, the equilibrium conversion of isobutylene is equal to approximately 0.95 under the conditions described in Example 1.

Data such as that in Table I (percent conversion of isobutylene versus time) is used mathematically to plot the rate of depletion of isobutylene, which is equal to the rate of formation of MTBE, versus Co(1−X−KX). The slope of such a plot is the forward rate constant ($k_{FOR}$) at a given temperature, and is a good indicator of the catalyst's kinetic properties.

The kinetic results from a series of MTBE preparation reactions are summarized in Table II. All reactions were carried out following the procedure described in Example 1. All of the catalysts tested were initially regenerated with sulfuric acid, rinsed and dried prior to use.

TABLE II

| Catalyst | Pore volume* cm$^3$/g | $k_{FOR}$ @ 70°C |
|---|---|---|
| Duolite EXP-A | 0.25 | 1.92 |
| Duolite C-265 | 0.30 | 1.80 |
| Duolite C-26H | 0.31 | 2.00 |
| Dowex MSC-1 | 0.31 | 1.87 |
| Amberlyst 15 | 0.42 | 1.87 |
| Duolite ES-276-A | 0.57 | 2.95 |
| Duolite ES-276-B | 0.63 | 2.64 |
| Duolite ES-276-C | 0.63 | 2.79 |
| Duolite ES-276-D | 0.63 | 2.67 |
| Duolite ES-276-E | 0.67 | 3.11 |
| Imac SPC-118 | 0.72 | 1.40 |
| Duolite EXP-B | 0.92 | 2.00 |

(*Determined by mercury intrusion porosimeter using standard procedures with dry resin in the hydrogen form)

As can be readily seen from the data in Table II, maintaining the pore volume of the catalyst within the specified ranges results in an increased reaction rate ($k_{FOR}$), on the order of 40 to 65 percent. Such a dramatic improvement in reaction kinetics could not have been predicted from the performance of the standard ion exchange catalysts.

Example 3

Preparation of tert-butyl alcohol

The general procedure set forth in Example 1 was followed to produce tert-butyl alcohol (TBA) using a prior art catalyst and catalysts of the invention. The reactor was charged with 327 g deionized water and 1660 g ethylene glycol monobutyl ether (butyl Cellosolve). Next 454 g of $C_4$ mixture containing 45 percent isobutylene, 20 percent butene-1 and 35 percent isobutane was weighed in. The dry equivalent of 119 ml of water expanded catalyst was introduced, and the reactor was operated isothermally at 90°C throughout the reaction.

The reaction rate constant for each catalyst is set forth in Table III. The constants were derived using an equilibrium conversion of isobutylene ($X_{eq}$) of 0.88 under the described conditions.

TABLE III

| Catalyst | $k_{FOR}$ (hr$^{-1}$) |
|---|---|
| Amberlyst 15 | 1.69 |
| Duolite ES-276-C | 2.16 |
| Duolite ES-276-E | 2.18 |

The equilibrium conversion of the olefin in etherification-alcoholification reactions is a function of temperature and reactant composition, such as stoichiometric ratio and quantity of inerts. The equilibrium constant is not dependent on the catalyst per se, which means that an improved catalyst increases the forward rate constant and also increases the reverse rate constant to the same extent. Use of an improved catalyst is equally beneficial to the reverse reactions (de-etherification and dehydration), although such reactions are typically run at operating, conditions which maximize the reverse rate constant and minimize

the forward rate constant. The instant invention contemplates the use of improved catalysts in both forward and reverse etherification-alcoholification reactions.

**Claims**

1. A process of preparation of ethers and/or alcohols from olefins or of conversion of ethers and/or alcohols to olefins, the olefins having a double bond attached to a tertiary carbon atom, in the presence of a sulphonated strong acid ion exchange resin catalyst, characterized in that the cation exchange resin catalyst has a pore volume in the range from 0.50 to 0.70 $cm^3/g$.

2. A process according to claim 1, wherein the catalyst comprises a matrix of polystyrene cross-linked with divinylbenzene.

3. A process according to claim 1 or 2, wherein a reaction mixture comprising one or more of such olefins is reacted under such conditions that the product contains one or more ethers and/or alcohols.

4. A process according to any preceding claim, wherein a reaction mixture comprising isobutylene is etherified by reaction with methanol to form methyl tert-butyl ether.

5. A process according to any preceding claim, wherein a reaction mixture comprising isobutylene is reacted with water to form tert-butyl alcohol.

6. A process according to claim 1 or 2, wherein a reaction mixture comprising one or more ethers or alcohols is reacted under such conditions that the product contains one or more olefins.

**Patentansprüche**

1. Verfahren zur Herstellung von Ethern und/oder Alkoholen aus Olefinen oder zur Umwandlung von Ethern und/oder Alkoholen in Olefine, wobei die Olefine eine Doppelbindung, die mit einerm tertiären Kohlenstoffatom verknüpft ist, besitzen, in Gegenwart eines sulfonierten stark sauren Ionenaustauscherharzkatalysators, dadurch gekennzeichnet, daß der Kationenaustauscherharzkatalysator ein Porenvolumen im Bereich von 0,50 bis 0,70 $cm^3/g$ besitzt.

2. Verfahren nach Anspruch 1, dadurch gekennzeichnet, daß der Katalysator eine Matrix aus Polystyrol, die mit Divinylbenzol verknüpft ist, aufweist.

3. Verfahren nach Anspruch 1 oder 2, dadurch gekennzeichnet, daß eine Reaktionsmischung aus einem oder mehreren derartigen Olefinen unter solchen Bedingungen umgesetzt wird, daß das Produkt einen oder mehrere Ether oder Alkohle enthält.

4. Verfahren nach einem der vorhergehenden Ansprüche, dadurch gekennzeichnet, daß eine Reaktionsmischung aus Isobutylen durch Umsetzung mit Methanol unter Bildung von Methyl-tert.-butylether verethert wird.

5. Verfahren nach einem der vorhergehenden Ansprüche, dadurch gekennzeichnet, daß eine Reaktionsmischung aus Isobutylen mit Wasser unter Bildung von Tert.-butyl-alkohol umgesetzt wird.

6. Verfahren nach Anspruch 1 oder 2, dadurch gekennzeichnet, daß eine Reaktionsmischung aus einem oder mehreren Ethern oder Alkoholen unter solchen Bedingungen umgesetzt wird, daß das Produkt eines oder mehrere Olefine enthält.

**Revendications**

1. Un procédé de préparation d'éthers et/ou d'alcools à partir d'oléfines ou de conversion d'éthers et/ou d'alcools en oléfines, les oléfines ayant une double liaison fixée à un atome de carbone tertiaire, en présence d'une résine échangeuse d'ions catalytique fortement acide sulfonée, caractérisé en ce que la résine échangeuse de cations catalytique a un volume des pores dans la gamme de 0,50 à 0,70 $cm^3/g$.

2. Un procédé selon la revendication 1 dans lequel la catalyseur comprend une matrice de polystyrène réticulé avec du divinylbenzène.

3. Un procédé selon la revendication 1 ou 2 dans lequel un mélange réactionnel comprenant une ou plusieurs de ces oléfines est mis à réagir dans des conditions telles que le produit contienne un ou plusieurs éthers et/ou alcools.

4. Un procédé selon l'une quelconque des revendications précédentes dans lequel un mélange réactionnel comprenant de l'isobutylène est éthérifié par réaction avec du méthanol pour former l'éther méthyl-tert-butylique.

5. Un procédé selon l'une quelconque des revendications précédentes dans lequel un mélange réactionnel comprenant de l'isobutylène est mis à réagir avec de l'eau pour former de l'alcool tert-butylique.

6. Un procédé selon la revendication 1 ou 2 dans lequel un mélange réactionnel comprenant un ou plusieurs éthers ou alcools est mis à réagir dans des conditions telles que le produit contienne un ou plusieurs oléfines.